# EUROPÄISCHE PATENTSCHRIFT

(11) **EP 0 500 565 B1**
(45) Veröffentlichungstag und Bekanntmachung des Hinweises auf die Patenterteilung: **18.05.1994**
(21) Anmeldenummer: 90915405.6
(22) Anmeldetag: 30.10.1990
(51) Int. Cl.: A61B 17/60

(54) **VORRICHTUNG ZUM FIXIEREN EINES KNOCHENS**
DEVICE FOR FIXING A BONE
DISPOSITIF POUR FIXER UN OS

(30) Priorität: 31.10.1989 DE 3936182; 30.04.1990 DE 4013822
(43) Veröffentlichungstag der Anmeldung: 02.09.1992
(73) Patentinhaber: FISCHER, Jürgen, Dr. med., D-69488 Birkenau (DE)
(72) Erfinder: FISCHER, Jürgen, Dr. med., D-69488 Birkenau (DE)
(74) Vertreter: Quermann, Helmut, Dipl.-Ing.
(86) Internationale Anmeldenummer: DE9000826
(87) Internationale Veröffentlichungsnummer: WO9106253

(56) Entgegenhaltungen:
- EP-A- 0 190 990
- EP-A- 0 194 187
- EP-A- 0 240 376
- DE-U- 8 717 097
- GB-A- 2 031 731
- US-A- 2 055 024

## Beschreibung

Die Erfindung betrifft eine Vorrichtung zum Fixieren eines Knochens, insbesondere eines menschlichen Bein- bzw. Armknochens mit folgenden Merkmalen:
- mindestens zwei beabstandet zueinander, senkrecht zur Knochenhauptachse anordbare und um den Knochen legbare Ringe,
- auf jeden Ring über dessen Umfang verteilt anordbare und mit diesem durch Klemmung fest verbindbare Drahtklammern, zum Halten von den Knochen in Art eines Kreuzes durchsetzenden Fixationsdrähten,
- auf jedem Ring über dessen Umfang verteilt anordbare und mit diesem durch Klemmung fest verbindbare Klammerelemente, zur gelenkigen Aufnahme von die Ringe verbindenden, längenveränderlichen Verbindungselementen,
- mindestens eine mit einem der Ringe durch Klemmung fest verbindbare Fixationsklammer, wobei diese einen im wesentlichen in der Knochenhauptachse ausgerichteten Stab aufnimmt, dessen freies Ende mit einer Fixationsvorrichtung für einen senkrecht zur Knochenhauptachse orientierten, den Knochen haltenden Lenkdraht versehen ist.

Eine derartige Vorrichtung zum Fixieren eines Knochens ist aus der EP-A-0.240.376 bekannt. Bei dieser weist der jeweilige Ring im wesentlichen Kreisquerschnitt auf, mit abgeflacht gestalteten, gegenüberliegenden Bereichen, die im wesentlichen senkrecht zur Knochenhauptachse angeordnet sind. Das jeweilige der Aufnahme eines Endes eines Verbindungselementes dienende Klammerelement ist durch zwei Backenpaare gebildet, die als separate Bauteile konzipiert sind. Jedes Backenteil weist zwei Backen auf, wobei jeder Backen im Bereich eines Endes mit einem Durchgangsloch und im Bereich des anderen Endes mit einer konkaven, sich nahezu über einen Halbkreis erstreckenden Wölbung versehen ist. Die beiden Backen des einen Backenpaares umschließen den zugeordneten Ring im Bereich dessen äußeren und inneren konvexen Kontur, während die beiden Backen des anderen Backenpaares ein stabförmiges Ende des längenveränderlichen Verbindungselementes umgreifen. Eine Befestigungsschraube ist durch die Löcher der beiden Backenpaare gesteckt und es wird mittels einer auf das freie Ende der Schraube aufgeschraubten Mutter eine Vorspannung auf die beiden Backenpaare gebracht, die damit den zugeordneten Bereich des längenverändeflichen Verbindungselementes und des Ringes zwischen sich klemmen, das eine Backenpaar liegt dabei am anderen Backenpaar an. Die Längenveränderbarkeit des Verbindungselementes ergibt sich aufgrund des Umstandes, daß der jeweilige, im zugeordneten Backenpaar gehaltene Abschnitt des Verbindungselementes mit einem Innengewinde versehen ist, wobei in die Gewindeabschnitte gegenüberliegender Verbindungsteile des Verbindungselementes ein Bestandteil des Verbindungselementes bildendes Stabteil mit gegenläufigen Außengewinde einschraubbar und mittels Kontermuttern feststellbar ist. - Soll bei der beschriebenen Vorrichtung das längenveränderliche Verbindungselement demontiert und Insbesondere durch ein anderes Verbindungselement ersetzt werden, führt dies wegen der Befestigung der beiden zugeordneten Backenpaare am Verbindungsteil bzw. am Ring mittels einer Befestigungsschraube dazu, daß auch die Verbindung des Backenpaares zum Ring gelöst wird und die Gefahr besteht, daß sich die Position des Verbindungselementes zum Ring verändert, was nicht gewünscht ist.

Aus der US-A-2,055,024 ist eine Vorrichtung zum Fixieren eines Knochens bekannt, bei der die Ringe verbindende längenveränderliche Verbindungselemente in definierte Aufnahmen der beiden Ringe einsetzbar und mit diesen befestigbar sind. Die Verbindungselemente können damit nicht frei auf dem Umfang der Ringe positioniert werden. Das jeweilige Verbindungselement weist eine in eine von vielen Ausnehmungen im einen Ring einsteckbare Stange mit Außengewinde auf, auf die eine Mutter aufgeschraubt ist. Das über diese hinausstehende Ende der Spindel ragt in eine Ausnehmung einer Hülse, die auf der der Spindel abgewandten Seite mit einem Kugelansatz versehen ist. Ein mit dem anderen Ring befestigbares Aufnahmeteil für den Kugelansatz dient dem Verspannen des Kugelansatzes gegen diesen anderen Ring, auf dessen der Stange abgewandten Seite. Montiert wird die Hülse, indem sie durch Ausnehmung im anderen Ring über die Stange geschoben wird. Wegen der Gestaltung des längenveränderlichen Verbindungselementes kann dieses nur dann ausgetauscht werden, wenn das Aufnahmeteil vom zugeordneten Ring entfernt wird.

Vorrichtungen zum Fixieren eines Knochens sind ferner aus der EP-A-0.190.990 und der DE-U-87 17 097 bekannt.

Es ist Aufgabe der vorliegenden Erfindung, eine Vorrichtung zum Fixieren eines Knochens, insbesondere eines menschlichen Bein- oder Armknochens zu schaffen, mit der die Fixierung mit einer geringen Anzahl erforderlicher Bauteile bei einfacher Handhabung sichergestellt ist.

Die erfindungsgemäße Vorrichtung zum Fixieren eines Knochens ist, ausgehend von der nach der eingangs genannten Art, gekennzeichnet durch folgende Merkmale:
- das jeweilige Verbindungselement ist durch ein Spannschloß und zwei in dieses eingeschraubte Spindeln, deren jeweils freies Ende einen Kugelansatz aufweist, gebildet, wobei der jeweilige Kugelansatz mit einer Kugelaufnahme des jeweiligen Klammerelementes zusammenwirkt,
- das jeweilige Klammerelement weist ein U-förmig ausgebildetes Backenteil und ein mit der Kugelaufnahme versehenes Aufnahmeteil auf,
- der Abstand der beiden Schenkel des Backenteils entspricht der Stärke des zugeordneten Ringes in dessen axialer Erstreckung und es sind die beiden Schenkel im Bereich deren freien Enden miteinander verschraubt,
- das Aufnahmeteil ist in der Knochenhauptachse geteilt ausgebildet, wobei eine der Aufnahmeteilhälften eine Baueinheit mit dem Backenteil bildet und mit der anderen Aufnahmeteilhälfte verschraubt ist, ferner weisen die Aufnahmeteilhälften auf den einander zugewandten Seiten die Kugelaufnahme bildende Kugelhalbschalen auf, mit einer auf den anderen Ring zugerichteten Ausnehmung zur Aufnahme der zugeordneten Kugel und zum Durchtritt der zugeordneten Spindel des jeweiligen längenveränderlichen Verbindungselementes.

Die erfindungsgemäße Ausbildung der Vorrichtung begünstigt die Fixierung des Knochens bei einfacher Handhabung und geringer Anzahl erforderlicher Bauteile. Die besonders einfache bauliche Gestaltung der Vorrichtung gestattet es, sie weithend in Einhand-Arbeitsweise zu montieren, d.h. es kommt der Operateur beim Zusammenmontieren der Vorrichtung im wesentlichen mit einer Hand für den Montagevorgang aus, während die andere Hand im wesentlichen Haltefunktionen übemimmt.

So kommt die erfindungsgemäße Vorrichtung grundsätzlich mit zwei Ringen aus, die nicht nur der Befestigung der Drahtkreuze und der längenveränderlichen Verbindungselemente dienen, sondern auch des mindestens einen Lenkdrahtes. Dessen ungeachtet können natürlich auch mehrere Ringe über Verbindungselemente miteinander verbunden werden und auch mehrere Lenkdrähte gleichen oder unterschiedlichen Ringen zugeordnet werden. Die Verbindung der die Fixationsdrähte aufnehmenden Drahtklammem, ferner der die längenveränderlichen Verbindungselemente aufnehmenden Klammerelemente und der Fixationsklammer für den Lenkdraht mit den Ringen erfolgt durch einfaches Klemmen, womit einerseits eine sichere Befestigung der genannten Bauteile am jeweiligen Ring sichergestellt ist, aber dessen ungeachtet die Bauteile sich vor dem Klemmen über die Circumferenz des Ringes verschieben lassen und somit auf einfache Art und Weise die gewünschte Anordnung des Fixationsdrahtes, des längenveränderlichen Verbindungselementes oder des Lenkdrahtes ermöglichen.

Der Abstand der Ringe kann auf einfache Art und Weise durch die als Spannschlösser mit den Spindeln ausgebildeten, längenveränderlichen Verbindungselemente variiert werden, die Kugelansätze der Spindeln ermöglichen im Zusammenwirken mit den Kugelaufnahmen des jeweiligen Klammerelementes eine relativ freie zug- und druckkraftübertragende Verschwenkbarkeit der Verbindungselemente zu den Ringen, während sich die eigentliche Längenveränderung durch Drehen des Spannschlosses bewerkstelligen läßt. Beidseitig des jeweiligen Spannschlosses angeordnete Kontermuttem stellen die vorgegebene Länge des Verbindungselementes sicher, durch die bei gewünschter Position der Ringe zueinander erzeugte Preßverbindung von Kugelansatz der Spindel und Kugelaufnahme des jeweiligen Klammerelementes läßt sich das Verbindungselement auch klammerelementseitig festlegen.

Von besonderer Bedeutung ist, daß bei dem jeweiligen Klammerelement eine der Aufnahmeteilhälften eine Baueinheit mit dem Backenteil bildet und mit der anderen Aufnahmeteilhälfte verschraubbar ist, wobei die Aufnahmeteilhälften auf den einander zugewandten Seiten die Kugelhalbschalen aufweisen. Beim Anbringen des längenveränderlichen Verbindungselementes zwischen zwei Ringen kann dieses zunächst mit der Kugel in die backenteilseitige Aufnahmeteilhälfte eingelegt und dann die andere Aufnahmeteilhälfte locker mit dieser verschraubt werden, anschließend wird das Backenteil bevorzugt von innen auf den Ring aufgesteckt und dessen beiden Schenkel mittels der Schraube lose verbunden, so daß das Backenteil auf der Circumferenz des Ringes beweglich und das längenveränderliche Verbindungselement nach wie vor schwenkbar ist. Erst wenn die Position des Verbindungselementes zum anderen Ring festliegt, insbesondere dessen Länge, kann durch Anziehen der Schrauben das Backenteil auf dem Ring und die Kugel des Verbindungselementes im Aufnahmeteil endgültig fixiert werden, wodurch das Aufnahmeteil wegen der die Kugel umgebenden Hinterschneidung im Bereich dessen Öffnung diese Kugel und damit das Verbindungselement zug- und druckkraftübertragend im Sinne der Längserstreckung des Verbindungselementes fixiert.

Diese Gestaltung ermöglicht es auch, das längenveränderliche Verbindungselement auf einfache Art und Weise gegen ein anderes Verbindungselement auszutauschen, ohne daß die Verbindung der Backenteile zum Ring gelöst werden muß. Das neu eingesetzte Verbindungselement befindet sich damit in der gleichen Wirkstellung wie das vorherige Verbindungselement. Dieser Vorteil ermöglicht es, Sätze von Verbindungselementen in geringen Längenstufenabständen vorzuhalten, mit der Konsequenz, daß die Verbindungselemente so kurz wie möglich sind und stattdessen öfter ausgewechseit werden.

Bevorzugt sind die bei der Vorrichtung Verwendung findenden Schrauben als Innensechskantschrauben ausgebildet Die Verwendung einer Innensechskantschraube birgt den Vorteil in sich, daß beim Arbeiten an der Extremität ein Abrutschen des Sechskantschlüssels aus der Ausnehmung der Innensechskantschraube gegen die Extremität weitgehend ausgeschlossen ist, jedenfalls ist die Verietzungsgefahr wesentlich geringer als bei der Verwendung einer üblichen Kopfschraube, die mittels eines Schraubenschlüssels angezogen werden muß.

Bei der erfindungsgemäßen Vorrichtung zum Fixieren eines Knochens ermöglicht des weiteren der Lenkdraht eine Ausrichtung des Knochens beabstandet zu dessen ringseitigen Drahtkreuzfixationsstellen. Die Fixationsklammer kann vor deren Klemmung über die Cicumferenz des Ringes verschoben werden und gibt nach deren Klemmung die grundsätzliche Ringposition vor, während der mit der Fixationsklammer verbundene Stab den Abstand des Lenkdrahtes vom jeweiligen zugeordneten Ring definiert. Die eigentliche Fixationsvorrichtung hält den im wesentlichen parallel zur zugeordneten Ringebene angeordneten, ausschließlich Zugkräfte übertragenden Lenkdraht, dessen der Fixationsvorrichtung abgewandtes Ende eine im Wirkstadium des Lenkdrahtes an der abgewandten Fläche des Knochens anliegende Verdickung, bevorzugt eine solche in Form einer Olive, aufweist.

Zweckmäßig ist der jeweilige Ring mit einem rechteckigen Querschnitt, gegebenenfalls mit abgerundeten Ecken, versehen und dessen Hauptfläche im wesentlichen senkrecht zur Knochenhauptachse angeordnet. Eine derartige Gestaltung des Ringes schafft große Klemmflächen für die Drahtklammern, die Klammerelemente und die Fixationsklammer in einer Ebene senkrecht zur Knochenhauptachse, somit in der Wirkrichtung der Fixationsdrähte und des Lenkdrahtes, andererseits große kraftübertragende Flächen für die zwischen den Ringen angeordneten, längenveränderlichen Verbindungselemente. Es besteht aber gleichfalls die Möglichkeit, die Ringe als Zylinderringe auszubilden, das heißt so, daß diese im wesentlichen rechteckigen Querschnitt aufweisen und deren Hauptflächen im wesentlichen in der Knochenhauptachse angeordnet sind. Der Vorteil der Zylinderringe ist darin zu sehen, daß bei größtmöglichem Abstand voneinander jeder stirnseitigen Ringebene ein Drahtkreuz zugeordnet werden kann und damit eine optimierte Zweietagenfixation des Knochens mittels eines einzigen Ringes möglich ist. Dessen ungeachtet kann auch bei einem flach ausgebildeten Ring eine Zweietagenfixation des Knochens bewerkstelligt werden, wegen der flachen Ausbildung des jeweiligen Ringes in Richtung der Knochenhauptachse sind die beiden dem Ring zugeordneten Drahtkreuze allerdings nur geringfügig beabstandet. Die Ringe selbst können unterschiedlichen Innendurchmesser bei im wesentlichen gleicher axialer und radialer Erstreckung aufweisen. Hierdurch besteht die Möglichkeit, die Ringgröße optimal auf die Außenabmessung der interessierenden Extremität abzustimmen. Die Ringe bestehen zweckmäβig aus einem solchen Kunststoffmaterial, das Röntgenstrahlen durchläßt und damit eine verbesserte Röntgendiagnostik bei angebrachter Vorrichtung ermöglicht, abgesehen von dem verbesserten Tragekomfort infolge des relativ geringen Gewichtes der Ringe. Zusätzlich können die Ringe zumindest in Teilumfangsbereichen mit einer Riffelung versehen sein, um so die Klemmung zwischen den Drahtklammern, den Klammerelementen und der Fixationsklammer zum jeweiligen Ring zu optimieren. Die Riffelung kann sowohl an den Stirnflächen als auch den Innen- oder Außenflächen vorgesehen sein. Durch eine Querschnittsmodifikation, durch eine Nutbildung an den Stirnflächen bzw. dem inneren oder äußeren Bereich des jeweiligen Ringes kann eine zusätzliche stabile dreidimensionale Fixation erfolgen. Die Ringe können mehrteilig, insbesondere zweiteilig ausgebildet sein, um sie so möglichst eng um die interessierende Extremität führen zu können.

In aller Regel wird jeder Ring mit vier Drahtklammern versehen, die jeweils um einen Winkel von 90° zueinander versetzt am Ring befestigt sind und das jeweilige Drahtkreuz halten. Üblicherweise werden auch vier Klammerelemente pro Ring vorgesehen, um die Ringe über die längenveränderlichen Verbindungselemente sowohl kippstabil als auch einfach manipulierbar zuordnen zu können. Grundsätzlich reicht es allerdings aus, drei Verbindungsstangen vorzusehen, hinsichtlich der Fixationsdrähte besteht selbstverständlich auch die Möglichkeit, weitere Drahtklammern dem jeweiligen Ring zuzuordnen. Mit jedem Ring können ein oder mehrere Fixationsklammern verbunden sein, insbesondere zwei Fixationsklammern, womit zwei Lenkdrähte den Knochen beabstandet zu den Drahtkreuzen, insbesondere in der Nähe des Bruch- bzw. Trennbereiches halten, und dies zweckmäßig in einem Winkel von etwa 90°. Die genaue Positionierung der Lenkdrähte hängt primär von der Lage der Hauptgefäße und der Muskeln, somit den auf den zu behandelnden Knochen einwirkenden Biegekräften ab, die zusätzlich über die Lenkdrähte abgefangen werden können.

Die nachfolgenden Ausgestaltungen der Drahtklammern, Fixationsklammern, und der Fixationsvorrichtung für den jeweiligen Lenkdraht verdeutlichen weitere Varianten, die eine besonders einfache bauliche Gestaltung der Vorrichtung gestatten und es erlauben, die Vorrichtung auch bezüglich dieser Teile weitgehend in Einhand-Arbeitsweise zu montieren. So ist vorgesehen, daß die jeweilige Drahtklammer U-förmig ausgebildet ist und der Abstand der beiden Schenkel der Drahtklammer der Stärke des zugeordneten Rings in dessen axialer Erstreckung entspricht, sowie eine Innensechskantschraube die beiden Schenkel im Bereich deren freien Enden miteinander verbindet, wobei zwischen dem Schraubenkopf und dem zugeordneten Schenkel eine Klemmplatte zum Klemmen des dieser zugewandten Fixationsdrahtes zwischen der Klemmplatte und dem Schenkel vorgesehen ist, sowie die Klemmplatte von der Schraube durchsetzt wird. Die jeweilige Drahtklammer wird bevorzugt nach radial außen auf den Ring aufgesteckt, dann die Innensechskantschraube durch die Klemmplatte und die Schenkel der Drahtklammer hindurchgeführt und auf den freien Gewindeabschnitt der Innensechskantschraube eine Mutter aufgeschraubt. Der jeweilige durch den Knochen durchgeschraubte bzw. durchgestoßene Fixationsdraht wird zwischen die Klemmplatte und den zugeordneten Schenkel der Drahtklammer eingelegt, diese auf dem Ring wunschgemäß ausgerichtet, gespannt und anschließend die Innensechskantschraube mit einem üblichen abgewinkelten Sechskantschlüssel festgezogen. Vorteilhaft ist das zumindest eine spitze Ende des Fixationsdrahtes mit einem Gewinde versehen und ermöglicht es damit, diesen weitgehend schonend durch den Knochen zu führen. Üblicherweise erfolgt dieses durch eine drückende Drehbewegung eines angespitzten Drahtes mit der Folge einer erhöhten Erwärmung und Gewebebeschädigung des Knochens, das als Gewindespitze ausgebildete Ende fördert demgegenüber einen schneidenden Durchgang der Drahtspitze durch den Knochen. Das Spannen des jeweiligen Fixationsdrahtes erfolgt durch eine externe Spannvorrichtung, die radial außen auf die zugeordnete Drahtklammer und den Ring aufsetzbar ist.

Die vorstehende Ausbildung der Drahtklammer findet bevorzugt bei einem Ring Anwendung, dessen Hauptfläche im wesentlichen senkrecht zur Knochenhauptachse angeordnet ist.

- Die Fixationsklammer zum Halten des Lenkdrahtes sollte gleichfalls U-förmig ausgebildet sein und der Abstand der beiden Schenkel der Fixationsklammer der Stärke des zugeordneten Ringes in dessen axialer Erstreckung entsprechen, sowie eine Innensechskantschraube die beiden Schenkeln im Bereich der freien Enden miteinander verbinden. Die Fixationsklammer selbst kann beispielsweise im Bereich des die Schenkel verbindenden Steges mit einer Durchgangsbohrung in der Knochenhauptachse versehen sein, die der über seine gesamte Länge mit einem Gewinde versehene Stab durchsetzt, wobei zwei beidseitig der Bohrung angeordnete Muttern den Gewindestab mit der Fixationsklammer verbinden. Alternativ zu dieser nur in Längsrichtung des Stabes veränderlichen Positionierung von Stab und Fixationsklammer ist es auch denkbar, den Stab schwenkbar, jedoch festlegbar in der Fixationsklammer zu lagern. In diesem Fall sollte die Fixationsklammer entsprechend der Ausbildung des Klammerelementes zur Aufnahme des Stabes ein Aufnahmeteil aufweisen, das in der Knochenhauptachse geteilt ausgebildet ist, wobei eine der Aufnahmeteilhälften eine Baueinheit mit dem Klammerteil der Fixationsklammer bildet und mit der anderen Aufnahmeteilhälfte mittels zweier Innensechskantschrauben verbindbar ist, wobei ferner die Aufnahmeteilhälften auf den einander zugewandten Seiten Kugelaufnahmen bildende Kugelhalbschalen aufweisen, mit einer in der Knochenhauptachse verlaufenden Ausnehmung zur Aufnahme des eine Kugel aufnehmenden einen Endes des Stabes, dessen anderes Ende mit einem Gewindeabschnitt versehen ist. Unabhängig von der Lagerung der Stäbe in der jeweiligen Fixationsklammer wird es als bevorzugt angesehen, wenn auf den klammerfernen Gewindeabschnitt des Gewindestabes die Fixationsvorrichtung aufschraubbar ist, die aus einer auf den Gewindestab aufschraubbaren Lagerhülse, die einen Durchgang senkrecht zur Knochenhauptachse aufweist und einer den Durchgang durchsetzenden Gewindehülse zur Aufnahme des Lenkdrahtes, mit beidseitig der Lagerhülse angeordneten Verstellmuttern, sowie einer in die Lagerhülse einsetzbaren Konusklemmvorrichtung für den Lenkdraht, besteht. Die Lagerung der Fixationsvorrichtung auf dem Stab ermöglicht ein Ausrichten des Lenkdrahtes in einer Ebene senkrecht zum Stab und parallel zu dieser Ebene, unabhängig von der gegebenenfalls möglichen Schwenkung um den Lagerpunkt des Aufnahmeteiles der noch nicht festgelegten Fixationsklammer. Im übrigen kann auch der jeweilige Lenkdraht im Bereich seines spitzen Endes mit einem Gewinde versehen sein. Nachdem der Lenkdraht durch den Knochen gebohrt ist und dessen Verdickung am Knochen anliegt, wird der Lenkdraht in die Gewindehülse eingeführt und von der Konusklemmvorrichtung gehalten. Das Spannen des Lenkdrahtes erfolgt durch die beidseitig der Lagerhülse angeordneten Verstellmuttern.

Die vorbeschriebene Vorrichtung gestattet eine stabile dreidimensionale Knochenfixierung unter Verwendung von nur zwei Ringen, die in verschiedenen Eben um den Knochen gelegt werden und einer zusätzlichen volumensparenden Knochenfixation mit nachträglicher Möglichkeit zur Knochenpositionskorrektur. Diese weist einen in Längsrichtung des Hauptknochenfragmentes ausgerichteten einseitigen oder beidseitigen Fixationsstab auf, indem in verschiedenen Winkeln Lenkdrähte, die durch den Gesamtknochen oder einzelne Knochenteile transfixiert werden, befestigt werden können. Die Lenkdrähte können in zwei Ebenen bewegt werden, hierdurch besteht die Möglichkeit einer exakten Steuerung der Knochenbewegung sowie einer dreidimensionalen Ausrichtung des Knochens im Raum.

Weitere Merkmale der Erfindung sind in der Beschreibung der Figuren und in den Unteransprüchen dargestellt, wobei bemerkt wird, daß alle Einzelmerkmale und alle Kombinationen von Einzelmerkmalen erfindungswesentlich sind.

In den Figuren ist die Erfindung anhand einer bevorzugten Ausführungsform mit mehreren Detaillösungen beispielsweise dargestellt, ohne auf diese beschränkt zu sein. Es stellt dar:
- Figur 1: eine räumliche Ansicht der bevorzugten Ausführungsform der erfindungsgemäßen Vorrichtung zum Fixieren eines Knochens, dargestellt für den Fall der Kortikotomie für die Unterschenkel-Montage der Vorrichtung,
- Figur 2: eine Detailansicht eines der in Figur 1 gezeigten Ringe mit aufgesetzter Drahtklammer und fixiertem Fixationsdraht,
- Figur 3: eine Detailansicht des Ringes gemäß Figur 2 mit auf die Drahtklammer aufgesetztem Drahtspanner,
- Figur 4: eine Detailansicht eines der in Figur 1 gezeigten Ringe mit mit diesem verbundenem Klammerelement und vom Klammerelement aufgenommenem längenveränderlichen Verbindungselement,
- Figur 5: eine Darstellung eines der in Figur 1 gezeigten Ringe mit mit dem Ring verbundener Fixationsklammer und von dieser aufgenommener Fixationsvorrichtung mit Lenkdraht,
- Figuren 6 bis 14: Detaillösungen betreffend bestimmte Bauteile, die bei der erfindungsgemäßen Vorrichtung Verwendung finden können.

Die Notwendigkeit zur Knochenfixation ergibt sich aus unterschiedlichen Indikationen. Während zum einen in der Traumatologie die stabile Fixation der Knochenfragmente zur frühzeitigen Konsulidierung angestrebt wird, ist andererseits bei Knochenfehlstellung oder Fehlwachstum eine sichere dreidimensionale Knochenführung zur Korrektur erwünscht. Diesem Erfordernis wird die nachstehend beschriebene, für den Fall der Kortikotomie des Unterschenkels verwendete Fixationsvorrichtung gerecht.

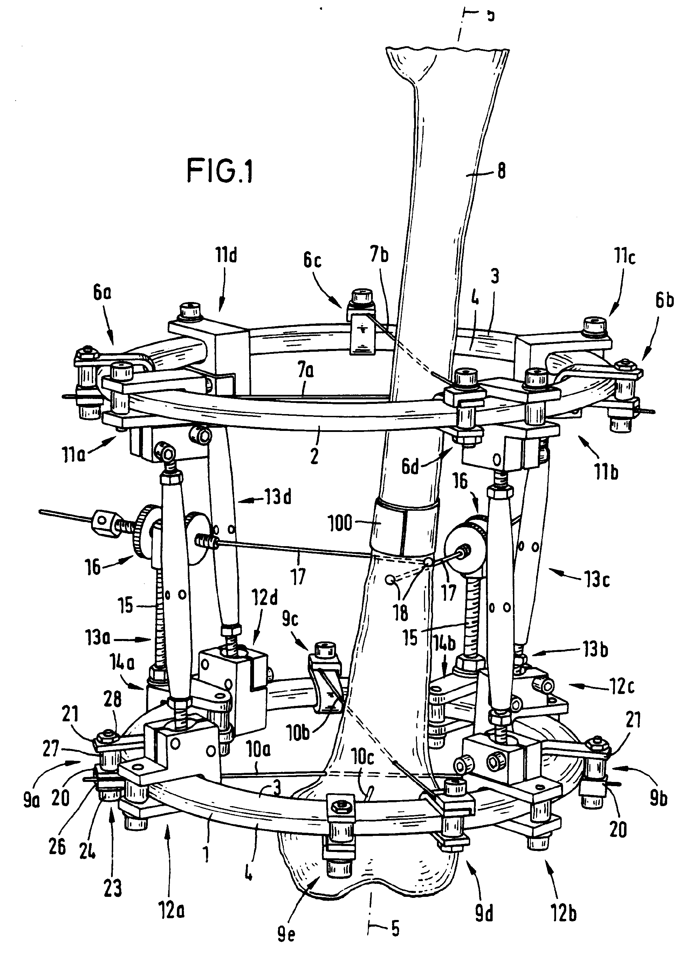

Figur 1 zeigt einen Unterschenkelknochen, um den im unteren Bereich ein erster Ring 1 und im Mittelbereich ein zweiter Ring 2 gelegt ist. Zwischen den Ringen 1 und 2 befindet sich der Kortikotomie-Bereich 100, das heißt der Bereich des Knochens, in dem dessen harte Knochenrinde eingekerbt ist, so daß er mittels der Vorrichtung verlängert werden kann. Entsprechend der äußeren Form des Unterschenkels sind die Abmessungen der Ringe 2 unterschiedlich gewählt, der untere Ring 1 weist einen geringeren Innendurchmesser, bei im wesentlichen gleicher axialer und radialer Erstreckung des Ringes auf als der obere Ring 2. Der Innendurchmesser des jeweiligen Ringes ist dabei näherungsweise so zu wählen, daß der Ring 1 bzw. 2 jeweils 3 cm Hautabstand aufweist. Die Ringe 1 und 2 weisen im wesentlichen rechteckigen Querschnitt auf, deren auf die Darstellung der Figur 1 bezogene obere Hauptflächen 3 und untere Hauptflächen 4 sind im wesentlichen senkrecht zur Knochenhauptachse 5 angeordnet. Mit dem oberen Ring 2 sind vier Drahtklammern 6a, 6b, 6c und 6d verbunden, wobei die Drahtklammer 6a diametral zur Drahtklammer 6b und die Drahtklammer 6c diametral zur Drahtklammer 6d angeordnet ist und die jeweiligen Drahtklammern 6a bis 6d jeweils um einen Winkel von etwa 90° auf der Circumferenz des Ringes 2 zueinander versetzt angeordnet sind. Die Drahtklammern 6a und 6b spannen zwischen sich unterhalb des Ringes 2 einen ersten Kirschner-Draht 7a, der den Oberschenkelknochen 8 durchsetzt, entsprechend spannen die Drahtklammern 6c und 6d zwischen sich oberhalb des Ringes 2 einen zweiten Kirschner-Draht 7b, der gleichfalls den Oberschenkelknochen 8 durchsetzt. Die beiden Kirschner-Drähte 7a und 7b bilden damit ein Drahtkreuz.

Entsprechend ist der untere Ring 1 mit vier Drahtklammern 9a, 9b, 9c und 9d versehen, von denen die Drahtklammern 9a und 9b diametral angeordnet sind und zwischen sich einen unterhalb des unteren Ringes 1 verlaufenden Kirschner-Draht 10a aufnehmen, der den Unterschenkelknochen 8 durchsetzt, sowie die Drahtklammern 9c und 9d diametral angeordnet sind, die zwischen sich ein Kirschner-Draht 10b aufnehmen, der oberhalb des unteren Ringes 1 geführt ist und den Unterschenkelknochen 8 durchsetzt. Die Drahtklammern 9a bis 9d sind auf der Circumferenz des unteren Ringes 1 jeweils um einen Winkel von etwa 90° versetzt angeordnet, so daß die beiden Kirschner-Drähte 10a und 10b ein Drahtkreuz bilden, das im übrigen, in der Knochenhauptachse 5 betrachtet, im wesentlichen mit dem aus den Kirschner-Drähten 7a und 7b gebildeten Drahtkreuz des oberen Ringes 2 fluchtet. Zusätzlich weist der untere Ring 1 zwei weitere Drahtklammern auf, von denen nur die eine Drahtklammer 9e, die benachbart der Drahtklammer 9d zwischen dieser und der Drahtklammer 9a angeordnet ist, zu sehen ist, während die andere Drahtklammer, die benachbart der Drahtklammer 9c zwischen dieser und der Drahtklammer 9b angeordnet ist, durch den Unterschenkelknochen verdeckt ist. Die Drahtklammer 9e und die diametral zu dieser angeordnete verdeckte Drahtklammer nehmen zwischen sich einen weiteren, unterhalb des unteren Ringes 1 geführten Kirschner-Draht 10c auf, der den Unterschenkelknochen 8 durchsetzt.

Mit dem oberen Ring 2 sind des weiteren vier Klammerelemente 11a, 11b, 11c und 11d verbunden, die auf der Circumferenz des Ringes 2 jeweils um 90° zueinander versetzt angeordnet sind. Das Klammerelement 11a ist dabei im wesentlichen in der Mitte zwischen den Drahtklammern 6a und 6d angeordnet, das Klammerelement 11b entsprechend zwischen den Drahtklammern 6d und 6b, das Klammerelement llc entsprechend zwischen der Drahtklammer 6b und 6c und das Klammerelement 11d entsprechend zwischen den Drahtklammern 6c und 6a. Gemäß der Anordnung der Klammerelemente 11a bis 11d ist der untere Ring 1 mit Klammerelementen 12a bis 12d versehen. Das Klammerelement 12a befindet sich zwischen den Drahtklammern 9a und 9e, das Klammerelement 12b zwischen den Drahtklammern 9d und 9b, das Klammerelement 12c zwischen der Drahtklammer 9b und der in Figur 1 nicht sichtbaren, mit der Drahtklammer 9e zusammenwirkenden Drahtklammer, das Klammerelement 12d zwischen den Drahtklammern 9c und 9a. Das Klammerelement 11a und das Klammerelement 12a verbindet ein längenveränderliches Verbindungselement 13a und entsprechend die Klammerelemente 11b und 12b ein Verbindungselement 13b, die Klammerelemente llc und 12c ein Verbindungselement 13c und die Klammerelemente 11d und 12c ein Verbindungselement 13d. Die Längsachse der längenveränderlichen Verbindungselemente 13a bis 13d ist im wesentlichen parallel zur Knochenhauptachse 5 orientiert.

Schließlich sind mit dem unteren Ring 1 noch zwei Fixationsklammern 14a und 14b verbunden. Die Fixationsklammer 14a ist benachbart zum Klammerelement 12d zwischen diesem und der Drahtklammer 9a angeordnet, die Fixationsklammer 14b bezüglich dieser, um einen Winkel von etwa 90° auf der Circumferenz des Ringes 1 versetzt, benachbart zum Klammerelement 12c zwischen diesem und der Drahtklammer 9c. Mit der jeweiligen Fixationsklammer 14a bzw. 14b ist jeweils ein senkrecht zur Ebene des Ringes 1 und damit im wesentlichen in der Knochenhauptachse 5 orientierter Stab 15 verbunden, der sich zwischen den beiden Ringen 1 und 2 erstreckt und dessen freies Ende mit einer Fixationsvorrichtung 16 für einen senkrecht zur Knochenhauptachse 5 orientierten, den Knochen unterhalb des Kortikotomiebereiches 100 haltenden Lenkdraht 17 versehen ist, wobei das der Fixationsvorrichtung 16 abgewandte Ende des Lenkrades 17 mit einer Verdickung in Form einer Olive 18 versehen ist, die mit dem Unterschenkelknochen 8 in Anlage gelangt.

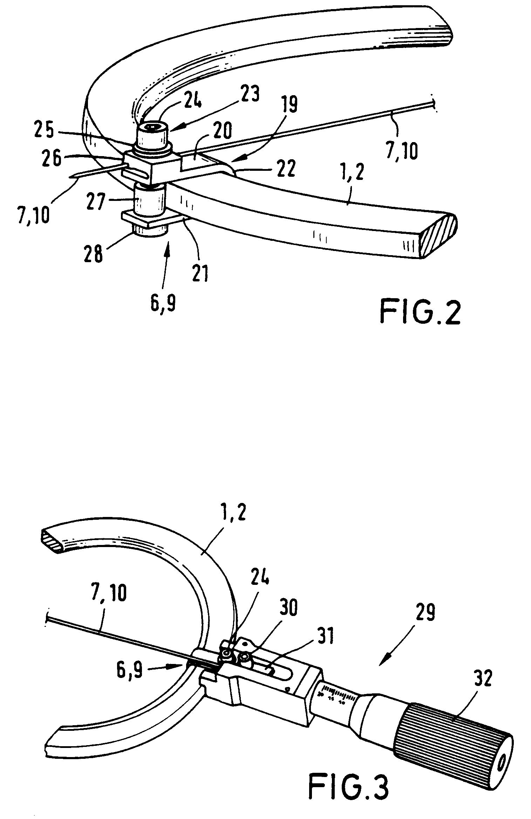

Nachfolgend seien unter weiterer Bezugnahme auf die Darstellung der Figuren 2 bis 6 die konstruktiven Details der erfindungsgemäßen Vorrichtung beschrieben, wobei die Drahtklammern allgemein mit der Bezugsziffer 6 bzw. 9, die Kirschner-Brähte mit der Bezugsziffer 7 bzw. 10, die Klammerelemente mit der Bezugsziffer 12, die längenveränderlichen Verbindungselemente mit der Bezugsziffer 13 und die Fixationsklammern mit der Bezugsziffer 14 bezeichnet werden.

Figur 2 verdeutlicht die Gestaltung einer Drahtklammer 6 bzw. 9 und deren Anordnung am Ring 1 bzw. 2 sowie die Fixierung des Kirschner-Drahtes 7 bzw. 10 an der Drahtklammer 6 bzw. 9. Die Drahtklammer weist einen aus einer rechteckigen, länglichen Platte U-förmig gebogenen Bügel 19 auf, wobei der Abstand der beiden Schenkel 20 und 21 des Bügels 19 der Stärke des Ringes in dessen axialer Erstreckung entspricht. Der die Schenkel 20 und 21 verbindende Steg 22 des Bügels 19 ist im wesentlichen halbkreisförmig ausgebildet und es weist der innere Rand des Ringes eine entsprechende Kontur auf, was auch für den äußeren Rand des Ringes gilt. Der Bügel 19 wird von innen auf den Ring aufgesteckt, wobei die freien Enden der Schenkel 20 und 21 über das Äußere des Ringes vorstehen und mit miteinander fluchtenden, nicht näher gezeigten Bohrungen versehen sind. Durch diese ist eine Innensechskantschraube 23 gesteckt, wobei zwischen dem Schenkel 20 und dem Kopf 24 der Innensechskantschraube 23, angrenzend an den Kopf 24, der Gewindeabschnitt der Innensechskantschraube 23 eine Unterlegscheibe 25 und dann eine Klemmplatte 26 durchsetzt, die so in Anlage mit der äußeren Hauptfläche des Schenkels 20 gelangt, wobei zwei an der Klemmplatte 26 angeordnete Ansätze den Schenkel 20 seitlich umgreifen und so die Klemmplatte drehsicher gehalten ist. Die Innensechskantschraube 23 durchsetzt mit ihrem Gewindeabschnitt eine zwischen den Schenkeln 20 und 21 angeordnete Hülse 27, deren axiale Erstreckung geringer ist als die axiale Erstreckung des Ringes. Auf der dem Kopf 24 abgewandten Seite des Schenkels 21 ist mit diesem eine mit der Bohrung in diesem Schenkel fluchtende Mutter 28 fest verbunden. Bei der Montage der Vorrichtung wird die jeweilige Drahtklammer 6 bzw. 9 mit ihren Bügel 19 auf den jeweiligen Ring 1 bzw. 2 gesteckt, zwischen die Schenkel 20 und 21 die Hülse 27 eingeführt, anschließend auf die Innensechskant- schraube 23 die Unterlegscheibe 25 und die Klemmplatte 26 aufgesteckt, die Innensechskantschraube 23 mit Unterlegscheibe 25 und Klemmplatte 26 durch die Bohrungen in den Schenkeln 20 und 21 sowie die Hülse 27 gesteckt und mit der Mutter 28 locker verschraubt. Es wird dann der jeweilige, den Unterschenkelknochen 8 durchsetzende Kirschner-Draht 7 bzw. 10 zwischen die Klemmplatte 26 und den Schenkel 20 eingeführt und die Innensechskantschraube 23 etwas weiter angezogen, so daß die beidseitig der Klemmplatte 26 angeordneten Ansätze in Seitenkontakt mit dem Schenkel 20 gelangen und damit die Klemmplatte 26 gegen Verdrehen sichern. Auch bei dieser Schraubposition der Innensechskantschraube 23 ist ein freies Ausrichten des Bügels 19 zum Ring und des Kirschner-Drahtes zwischen der Klemmplatte 26 und dem Schenkel 20 möglich. Nach dem Ausrichtvorgang und dem Spannen des Kirschner-Drahtes können im Einhandbetrieb, beispielsweise mittels eines Drehmomentschlüssels mit Ratsche die Drahtklammer und der Kirschner-Draht fixiert werden, wobei der Bügel 19 mit seinen beiden Schenkeln 20 und 21 den Ring zwischen sich klemmt, und die Klemmplatte 26 und der Schenkel 20 des Bügels 19 den Kirschner-Draht zwischen sich. Figur 3 zeigt einen auf den Ring 1 bzw. 2 mit Drahtklammer 6 bzw. 9 und KirschnerDraht 7 bzw. 10 aufgesetzten Drahtspanner 29, der so kalibriert ist, daß eine exakte Spannung des Kirschner-Drahtes erreicht wird. Der Kirschner-Draht wird in eine Öffnung am Drahtspanner 29 gesteckt und mit einer Innensechskantschraube 30 fixiert. Der Spanner steckt dabei mit seiner Gabelöffnung 31 auf der Draht- klammer. Wenn der Griff 32 des Drahtspanners 29 gedreht wird, erfolgt die Spannung auf den Kirschner-Draht. Ist die erforderliche Spannung erreicht, wird die Innensechskantschraube 23 der Drahtklammer mit der Drehmomentratsche angezogen und der Draht- spanner entfernt.

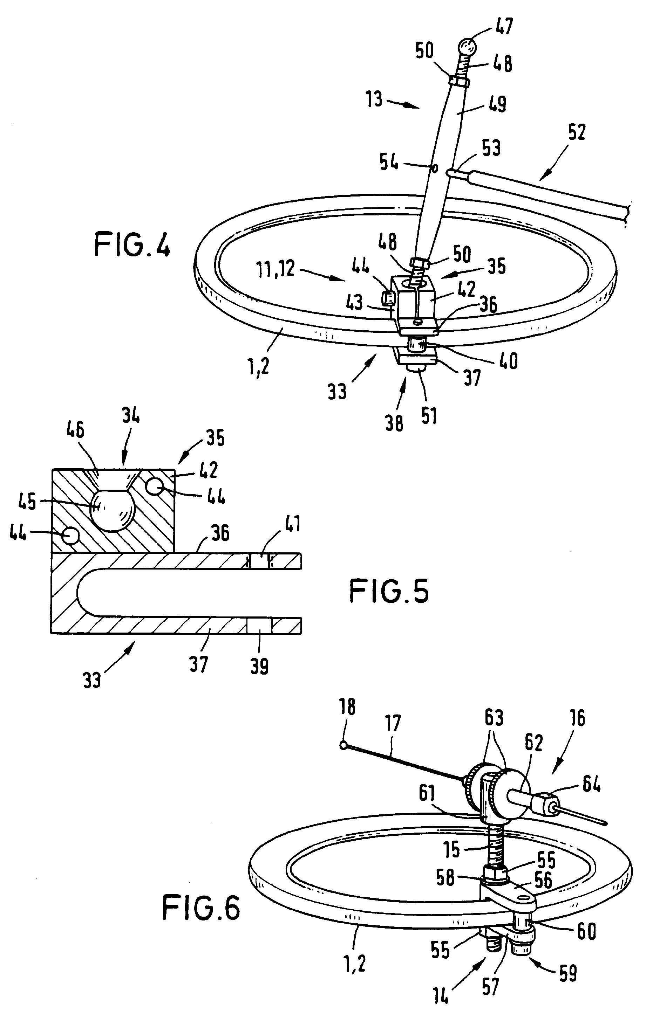

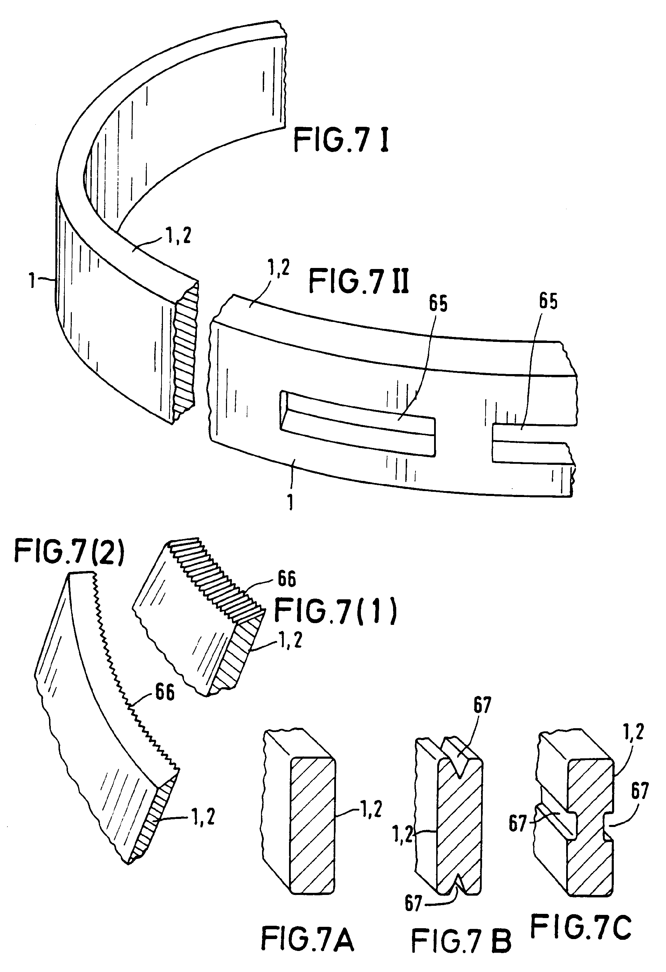

Figur 4 zeigt in einer Detaildarstellung die Verbindung eines Klammerelementes 11 bzw. 12 mit einem der Ringe 1 bzw. 2, sowie die Verbindung der Klammerelemente 11 bzw. 12 mit einem der längenveränderlichen Verbindungselemente 13. Wie dieser Figur und der Schnittdarstellung gemäß Figur 5 zu entnehmen ist, weist das Klammerelement ein U-förmig ausgebildetes Backenteil 33 und ein mit einer Kugelaufnahme 34 versehenes Aufnahmeteil 35 auf. Der Abstand der beiden Schenkel 36 und 37 des Backenteiles 33 entspricht dabei gemäß der Gestaltung der Drahtklammern 6 bzw. 9 der Stärke des zugeordneten Ringes in dessen axialer Erstreckung. Eine Innensechskantschraube 38 verbindet die beiden Schenkel 36 und 37 im Bereich deren freien Enden miteinander, indem deren Gewindeabschnitt eine Bohrung 39 im Schenkel 37 und eine der Hülse 27 entsprechende Hülse 40 durchsetzt und in eine Gewindebohrung 41 des Schenkels 36 eingeschraubt ist. Das Aufnahmeteil 35 ist in der Knochenhauptachse geteilt ausgebildet, wobei die in Figur 5 gezeigte eine Aufnahmeteilhälfte 42 eine Baueinheit mit dem Backenteil 33 bildet und mit der anderen Aufnahmeteilhälfte 43 mittels zweier Innensechskantschrauben 44 verbindbar ist. Jede der Aufnahmeteilhälften 42 und 43 weist auf den der jeweiligen anderen zugewandten Seite eine die Kugelaufnahme 34 bildende Kugelhalbschale 45 auf, mit einer auf den anderen Ring zugerichten Ausnehmung 46. Die Kugelaufnahme 34 und die Ausnehmung 46 dient der Aufnahme einer zugeordneten Kugel 47 sowie Spindel 48 des jeweiligen längenveränderlichen Verbindungselementes 13. In der Figur 4 ist die untere, mit dem Klammerelement verbundene Kugel nicht gezeigt, Anordnung von Spindel 48 und Kugel 47 ergeben sich aber entsprechend dem gezeigten anderen Ende des Verbindungselementes 13. Im Detail ist das Verbindungselement 13 als bekanntes Bauelement ausgebildet, das ein Spannschloß 49 und die beiden in dieses eingeschraubten Spindeln 48 aufweist, wobei eine der Spindeln Linksgewinde, die andere Spindel Rechtsgewinde aufweist, so daß bei einer Drehung des Spannschlosses 49 in einer Richtung sich die beiden Kugeln 47 voneinander wegbewegen, bei einer Drehung im anderen Drehsinne aufeinander zubewegen, was zu einer Längenvergrößerung bzw. Längenverkürzung des Verbindungselemen- tes 13 führt. Zusätzlich ist auf jede Spindel 48 eine Kontermutter 50 aufgeschraubt. Zur Montage von Klammerelement 11 bzw. 12 und längenveränderlichem Verbindungselement 13 wird bei abgenommener Aufnahmeteilhälfte 43 die zugeordnete Kugel 47 in die Kugelhalbschale 45 der Aufnahmeteilhälfte 42 eingelegt, anschließend die andere Aufnahmeteilhälfte 43 aufgelegt und die beiden Aufnahmeteilhälften 42 und 43 mittels der beiden Innensechskantschrauben 44 lose miteinander verschraubt. Dann wird das Backenteil 33 von innen auf den Ring 1 bzw. 2 aufgesteckt und die Innensechskantschraube 38 in die Gewindebohrung 41 eingeschraubt, wobei zweckmäßig unter den Kopf 51 der Innensechskantschraube 38 und den Schenkel 37 eine Unterlegscheibe gelegt wird. Auch die Innensechskantschraube 38 wird nur lose eingeschraubt, so daß ein Ausrichten des Klammerelementes 11 bzw. 12 und des Verbindungselementes 13 einschließlich dessen Längenanpassung möglich ist. Ist dies erfolgt, werden die Imbusschrauben 38 und 44 angezogen und auch die Kontermuttern 50 gegen das Spannschloß 49 verspannt, so daß sich das Klammerelement 11 bzw. 12 und das längenveränderliche Verbindungselement 13 zusammen mit dem Ring 1 bzw. 2 als starre Einheit darstellen. In der Figur 4 ist zusätzlich ein Stiftbolzenwerkzeug 52 gezeigt, dessen Stiftbolzen 53 in eine von mehreren mittigen Bohrungen 54 einsteckbar ist, um so das Spannschloß 49 drehen zu können.

Figur 6 zeigt die gleichfalls U-förmig ausgebildete Fixationsklammer 14, die entsprechend dem Backenteil 33 ausgebildet ist, mit dem Unterschied, daß der die Schenkel verbindende Steg eine größere Stärke aufweist und mit einer sich in Stegrichtung erstreckenden Bohrung versehen ist. Diese Bohrung durchsetzt der ein Gewinde aufweisende Stab 15, dieser ist mit der Fixationsklammer 14 mittels zweier auf den Gewindestab 15 aufgeschraubter Muttern 55 verbunden, wobei diese zwischen sich und den Schenkeln 56 bzw. 57 eine Unterlegscheibe 58 aufnehmen. Bei von innen auf den Ring 1 bzw. 2 aufgestecktem U-förmigen Bügel wird, wie zuvor zu den Figuren 4 und 5 beschrieben, eine Innensechskantschraube 59 auf deren Gewindeabschnitt gegebenenfalls auch eine Unterlegscheibe gesteckt ist, durch die Bohrung im Schenkel 57 gesteckt und durch eine entsprechend der Hülse 40 ausgebildete Hülse 60 in eine Gewindebohrung des Schenkels 56 eingeschraubt. Auf den klammerfernen Gewindeabschnitt des Gewindestabes 15 ist die Fixationsvorrichtung 16 aufgeschraubt, die ein Trägerelement 61 aufweist, das eine Sackbohrung mit Innengewinde besitzt, in das der Gewindestab 15 einschraubbar ist. Senkrecht zur Längsachse des Gewindestabes 15 durchsetzt das Trägerelement 61 eine Gewindebohrung, in die eine Gewindehülse 62 eingeschraubt ist. Zwei Konterscheiben 63 mit Außenrändelung sind beidseitig des Trägerelementes 61 auf die Gewindehülse 62 aufgeschraubt. Das zum Ringäußeren gewandte Ende der Gewindehülse 62 nimmt eine in die Durchgangsbohrung der Gewindehülse 62 einsetzbare Konusklemmvorrichtung 64 für den Lenkdraht 17 auf, dessen dem Ringinneren zugewandtes Ende mit der Olive 18 versehen ist. Auch die Fixationsklemme 14 wird erst nach dem Ausrichten der Fixationsvorrichtung durch Festschrauben der Innensechskantschraube 59 dauerhaft mit dem Ring 1 bzw. 2 verbunden. Dann wird durch Verdrehen der ringäußeren Rändelschraube 63 und damit eine entsprechende Bewegung der Gewindehülse 62 und der Konusklemmvorrichtung 64 mit dem Lenkdraht 47 der Knochen in Richtung des Ringäußeren ausgerichtet und die Fixationsvorrichtung 16 in der gewünschten Position mittels der der Olive 18 zugewandten Konterscheibe 63 festgelegt.

Die nachfolgenden Figuren stellen Detaillösungen dar:
Die Figur 7 bezieht sich auf die Ausbildung des Ringes 1 bzw. 2, der bevorzugt aus Kunststoff und zwar aus einem solchen, der röntgenstrahlendurchlässig ist, hergestellt ist. Entgegen der Ringvariante nach den Figuren 1 bis 6 ist dort der Ring 1 bzw. 2 bezüglich dessen Hauptflächen im wesentlichen in Knochenhauptachse 5 orientiert. Der Ring 1 bzw. 2 kann gleichmäßig aüsgebildet sein (I) oder auch senkrecht zur Außen-/Innenfläche angeordnete Schlitze 65 aufweisen (II). Ferner kann eine Riffelung 66 im Bereich deren Stirnfläche (1) oder der Innenfläche (2) vorgesehen sein. Es besteht darüber hinaus die Möglichkeit, beim Zylinderring 1 bzw. 2 unterschiedliche Querschnittsformen vorzusehen, die von einem rechteckigen Querschnitt (A) ausgehen, wobei sich die Lagerung der Drahtklammern 6 bzw. 9, der Klammerelemente 11 bzw. 12 und der Fixationsklammer 14 im Ring 1 bzw. 2 durch Nuten 67 im Bereich des oberen und unteren Randes des Ringes 1 bzw. 2 (B) oder des inneren und äußeren Randes (C) und eine entsprechende komplementäre Ausgestaltung der Drahtklammern, Klammerelemente und der Fixationsklammer optimieren läßt.

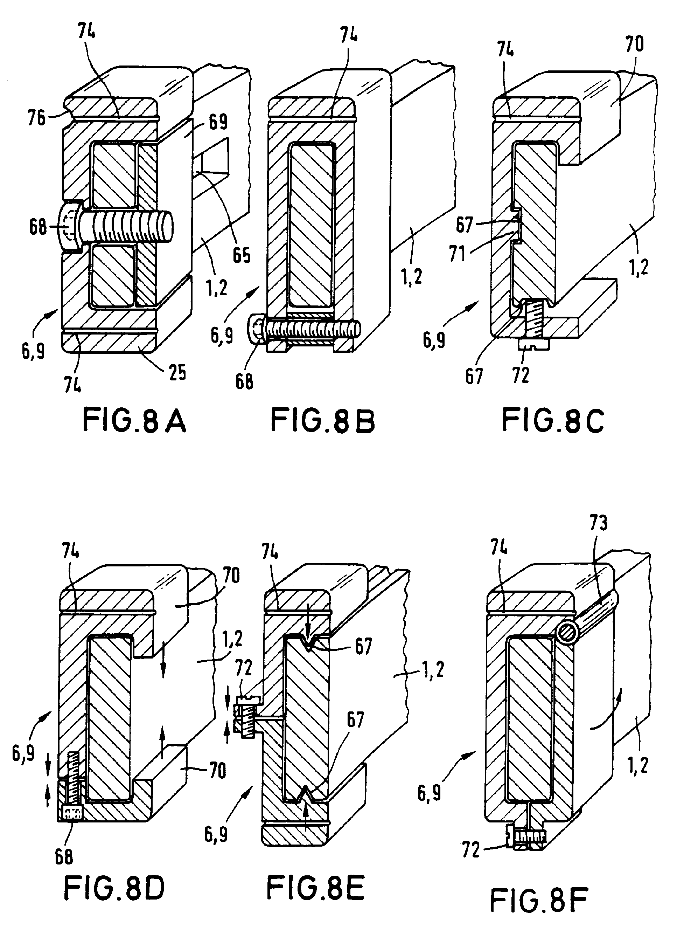

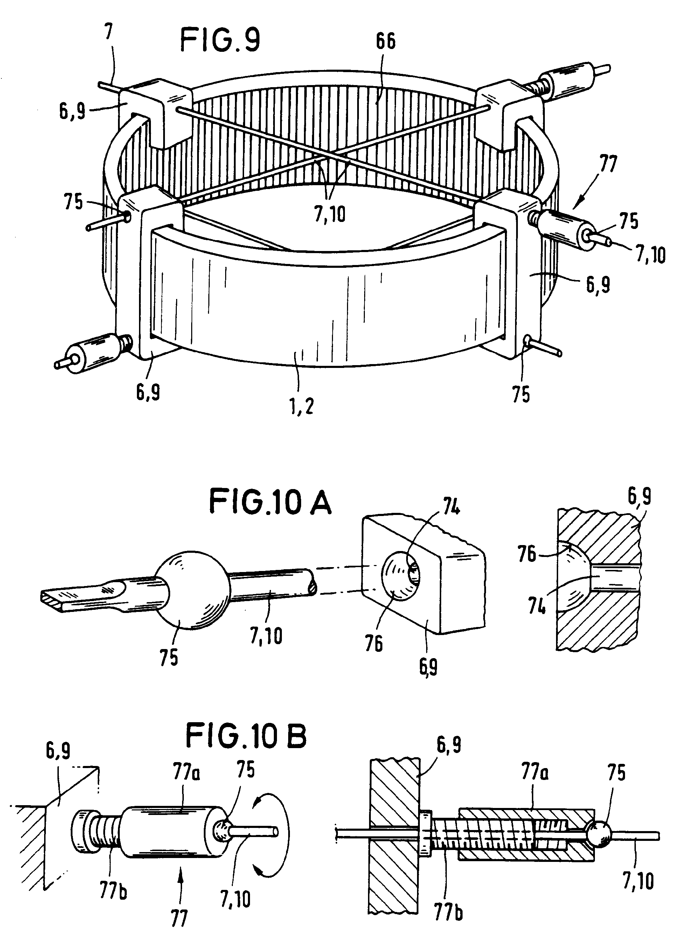

Figur 8 verdeutlicht weitere mögliche Ausgestaltungen der Drahtklammern 6 bzw. 9, wobei die Verbindung der Drahtklammern mit dem Ring 1 bzw. 2 entsprechend auch für die Klammerelemente 11 bzw. 12 oder die Fixationsklammer 14 gilt. So kann die jeweilige Drahtklammer, wie zuvor bereits beschrieben, U-förmig ausgebildet sein und den Ring, insbesondere einen zylinderförmig ausgebildeten Ring oben und unten umschließen, wobei eine Innensechskantschraube 68 den Schlitz 65 durchsetzt und mit einem rückwärtigen Paßstück 69 verschraubt ist, womit eine Klemmung zwischen Drahtklammer 6 bzw. 9 und Ring 1 bzw. 2 erfolgt (A). Bei einer weiteren U-förmigen Ausbildung der Drahtklammer kann vorgesehen sein, diese von oben über den Zylinderring zu stecken und die unten über den Zylinderring hinausstehenden Schenkelbereiche der Drahtklammer mittels einer Innensechskantschraube 68 zu spannen (B). Die Drahtklammer kann weiterhin mit einer Nase 70 oben in den Zylinderring eingehängt und zusätzlich im Bereich einer Erhöhung 71 in der Nut 67 geführt sein. In diesem Fall bietet es sich an, die Drahtklammer über eine in die untere Nut 67 eingreifende Schraube 72 zu verspannen (C). Eine weitere Modifikation sieht vor, die Drahtklammer zweiteilig auszubilden, jeweils mit einer oberen und unteren Nase 70 und die beiden Teile mittels einer Innensechskantschraube 68 zu verbinden und damit die Drahtklammer bezüglich des Zylinderringes zu verspannen (D). Die Verspannung der zweiteiligen Drahtklammer kann dabei auch im Bereich eines Flansches erfolgen und überdies eine zusätzliche Führung gemäß der Darstellung in Figur 7, dort Beispiel B vorgesehen sein (E). Schließlich könnte bei einer zweiteiligen Ausbildung der Drahtklammer ein Klammerteil mittels eines Scharnieres 73 am anderen angelenkt sein, wobei dort eine Verschraubung der beiden Drahtklammerteile miteinander vorgesehen ist (F). Bei den Ausführungsformen A und E ist von besonderer Bedeutung, daß die Drahtklammern sowohl oberhalb als auch unterhalb des Zylinderringes 1, 2 mit Durchgangsbohrungen 74 für die Kirschner-Drähte 7 versehen sind, die damit eine Zweietagenfixation der Drahtkreuze an einem Ring 1,2 ermöglichen, was in Figur 9 gezeigt ist.

Figur 10 zeigt ein Ende eines Kirschner-Drahtes 7 bzw. 10, der in Abwandlung zu der in den Figuren 1 bis 3 beschriebenen Ausführungsform mit einer kugelförmigen Verdickung 75 versehen ist und die mit einer entsprechenden Ausnehmung 76 der Drahtklammer, die einmal räumlich, im anderen Fall im Schnitt dargestellt ist, zusammenwirkt (A). Die Figur zeigt ferner einen Gewindespanner 77, mit dessen Schraubhülse 77a die gleichfalls entgegen der Ausführungsform nach den Figuren 1 bis 5 kugelförmige Verdickung 75 des anderen Endes des Kirschner-Drahtes 7 bzw. 10 zusammenwirkt und die auf einen Sockel 77b aufschraubbar ist (B).

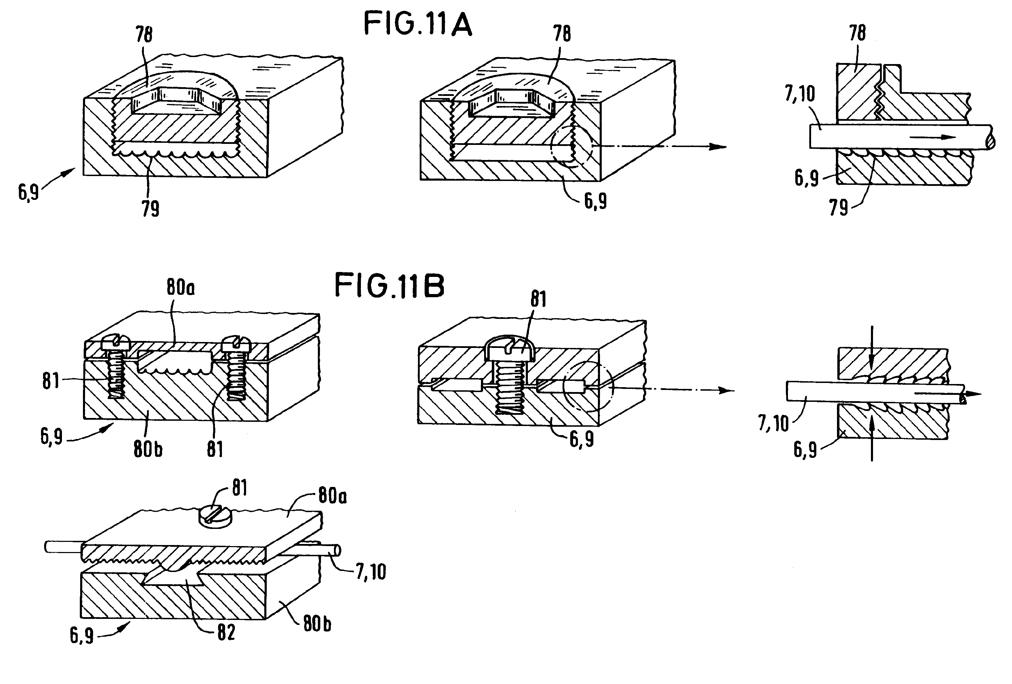

Figur 11 zeigt Varianten betreffend die Verspannmöglichkeit des Kirschner-Drahtes 7 bzw. 10. So kann die Pressung auf den Kirschner-Draht mittels eines in die Drahtklammer 6 bzw. 9 einschraubbaren Preßstückes 78 aufgebracht werden, wobei bei dieser Ausbildung entweder der diesem Reststück zugeordnete Boden 79 der Drahtklammer mit einer entgegen der Zugrichtung des Kirschner-Drahtes verlaufenden Längsriffelung versehen ist oder aber glatt, wobei hier jedoch dann die Durchgangsbohrung des Kirschner-Drahtes in der Drahtklammer mit der Riffelung versehen sein sollte, die überdies vorteilhaft teilweise in den Bodenbereich ragen sollte (A). Es besteht daneben die Möglichkeit, den Kirschner-Draht zwischen zwei separaten Platten 80a und 80b der Drahtklammer mittels Schrauben 81 zu verspannen, wobei auch in diesem Fall die einander zugeordneten Flächen der Platten 80a und 80b eine Längsriffelung aufweisen sollten bzw. ein Profil, insbesondere ein schwalbenschwanzförmiges Profil 82 (B).

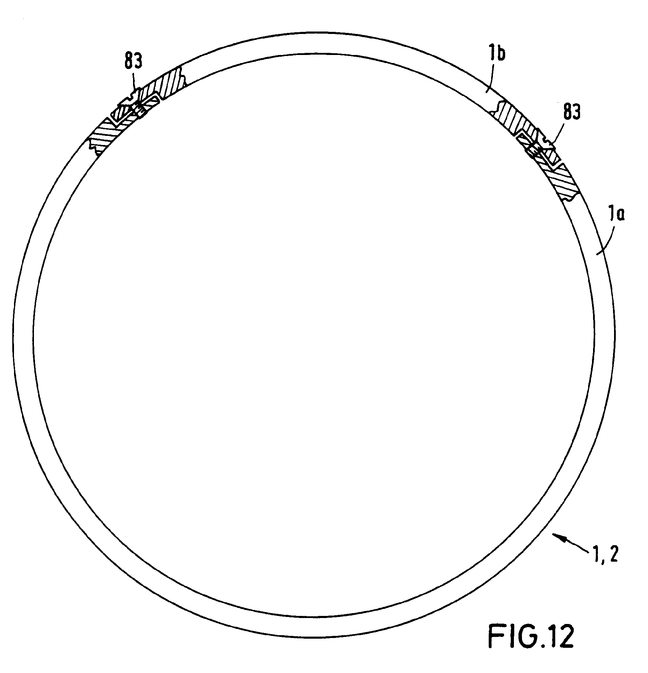

Figur 12 zeigt einen zweiteiligen Zylinderring 1 bzw. 2, der im Bereich eines Viertelkreises geschlitzt ist. Die beiden Ringsegmente la und 1b werden dort mittels zweier Schrauben 83 zum Gesamtring verbunden.

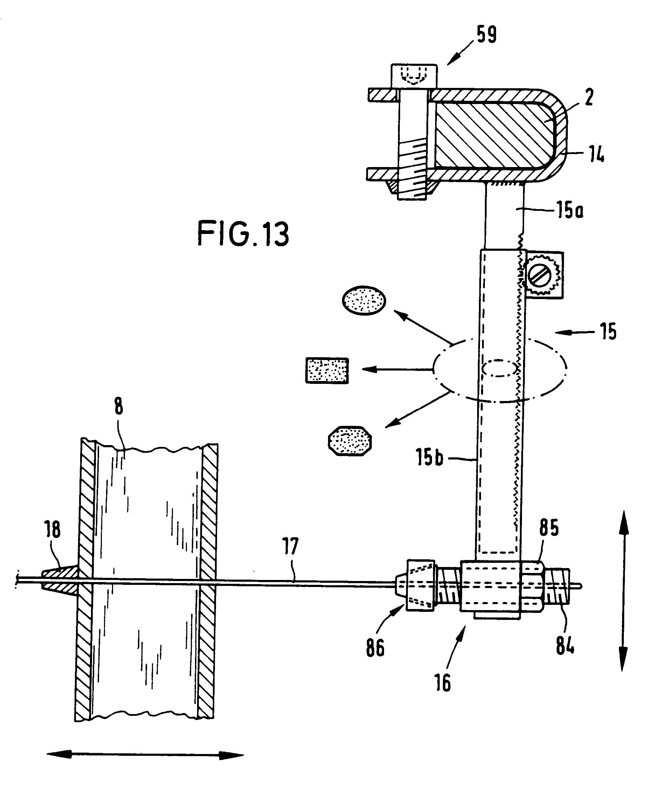

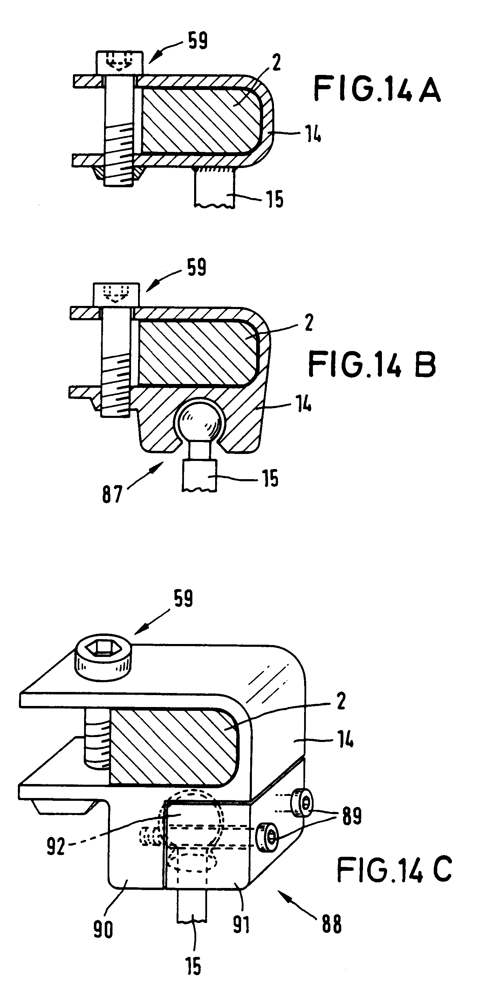

Die Figuren 13 und 14 verdeutlichen Detaillösungen betreffend die ergänzende stabilisierende Fixation des Knochens mittels der Fixationsklammern 14 und der Fixationsvorrichtung 16. Bei der Ausführungsform nach der Figur 13 ist der Stab 15 zweigeteilt, wobei ein Stabteil 15a fest mit der Fixationsklammer 14 verbunden ist. Die Länge des Stabes läßt sich durch ein zweites als Gegenstück passendes Stabteil 15b durch ein Schneckengewinde in der Längsachse des Knochens 8 verändern. Die Fixationsvorrichtung 16 erlaubt durch ein Gewinde 84 eine rechtwinklig zur Knochenachse gelegene Korrektur. Dies wird gewährleistet durch eine Überwurfmutter 85. Die Drahtfixation erfolgt durch eine zentrale Perforation der durch einen konusförmigen Klemmteil mit Überwurfmutter 86 fixiert wird. Figur 14 verdeutlicht die Fixation der längs zur Knochenachse angebrachten Fixationsmöglichkeiten. Diese kann, wie auch in Figur 13 dargestellt, auf der Fixationsklammer 14 durch eine feste stabile Verbindung mit dem Stab 15 vorgenommen werden (A). Alternativ kann eine in zwei Ebenen frei bewegliche Konusklemmvorrichtung durch eine im Schlüssel-Schloß-Prinzip vorgefertigte Vorrichtung 87 erfolgen (B). Ferner kann ein kugelförmiges Ende durch ein Zweibackensystem 88 mittels zweier Schrauben 89 fest stabil im Raum verankert werden. Dabei bildet eine der Aufnahmeteilhälften 90, 91 des Zweibackensystems 88 eine Baueinheit mit dem Klammerteil der Fixationsklammer 14, wobei die Aufnahmeteilhälften 90 bzw. 91 auf den einander zugewandten Seiten eine Kugelaufnahme bildende Kugelhalbschalen aufweisen, mit einer in der Knochenhauptachse 5 verlaufenden Ausnehmung des die Kugel 92 aufnehmenden einen Stabendes, dessen anderes Ende mit dem Gewindeabschnitt versehen ist.

### Bezugszeichenliste

- 1, 2: Ring
- 3, 4: Hauptfläche
- 5: Knochenhauptachse
- 6, 6a, 6b, 6c, 6d: Drahtklammer
- 7, 7a, 7b: Kirschner-Draht
- 8: Unterschenkelknochen
- 9, 9a, 9b, 9c, 9d, 9e: Drahtklammer
- 10, 10a, 10b, 10c: Kirschner-Draht
- 11, 11a, 11b, 11c, 11d: Klammerelement
- 12, 12a, 12b, 12c, 12d: Klammerelement
- 13, 13a, 13b, 13c, 13d: längenveränderliches Verbindungselement
- 14, 14a, 14b: Fixationsklammer
- 15: Stab
- 15a, 15b: Stabteil
- 16: Fixationsvorrichtung
- 17: Lenkdraht
- 18: Olive
- 19: Bügel
- 20, 21: Schenkel
- 22: Steg
- 23: Innensechskantschraube
- 24: Kopf
- 25: Unterlegscheibe
- 26: Klemmplatte
- 27: Hülse
- 28: Mutter
- 29: Drahtspanner
- 30: Innensechskantschraube
- 31: Gabelöffnung
- 32: Griff
- 33: Backenteil
- 34: Kugelaufnahme
- 35: Aufnahmeteil
- 36, 37: Schenkel
- 38: Innensechskantschraube
- 39: Bohrung
- 40: Hülse
- 41: Gewindebohrung
- 42, 43: Aufnahmeteilhälfte
- 44: Innensechskantschraube
- 45: Kugelhalbschale
- 46: Ausnehmung
- 47: Kugel
- 48: Spindel
- 49: Spannschloß
- 50: Kontermutter
- 51: Kopf
- 52: Stiftbolzenwerkzeug
- 53: Stiftbolzen
- 54: Bohrung
- 55: Mutter
- 56, 57: Schenkel
- 58: Unterlegscheibe
- 59: Innensechskantschraube
- 60: Hülse
- 61: Trägerelement
- 62: Gewindehülse
- 63: Konterscheibe
- 64: Konusklemmvorrichtung
- 65: Schlitz
- 66: Riffelung
- 67: Nut
- 68: Innensechskantschraube
- 69: Paßstück
- 70: Nase
- 71: Erhöhung
- 72: Schraube
- 73: Scharnier
- 74: Durchgangsbohrung
- 75: Verdickung
- 76: Ausnehmung
- 77: Gewindespanner
- 77a: Schraubhülse
- 77b: Sockel
- 78: Preßstück
- 79: Boden
- 89a, 80b: Platte
- 81: Schraube
- 82: Profil
- 83: Schraube
- 84: Gewinde
- 85: Überwurfmutter
- 86: Klemmkeil mit Überwurfmutter
- 87: Vorrichtung
- 88: Zwei-Backen-System
- 89: Schraube
- 90, 91: Aufnahmeteilhälfte
- 92: Kugel
- 100: Kortikotomiebereich

## Patentansprüche

1. Vorrichtung zum Fixieren eines Knochens, insbesondere eines menschlichen Bein- bzw. Armknochens, mit folgenden Merkmalen:
- mindestens zwei beabstandet zueinander, senkrecht zur Knochenhauptachse (5) anordbare und um den Knochen (8) legbare Ringe (1,2),
- auf jeden Ring (1, 2) über dessen Umfang verteilt anordbare und mit diesem durch Klemmung fest verbindbare Drahtklammern (6, 9) zum Halten von den Knochen (8) in Art eines Kreuzes durchsetzenden Fixationsdrähten (7, 10),
- auf jedem Ring (1, 2) über dessen Umfang verteilt anordbare und mit diesem durch Klemmung fest verbindbare Klammerelemente (11, 12) zur gelenkigen Aufnahme von die Ringe (1, 2) verbindenden längenveränderlichen Verbindungselementen (13),
- mindestens eine mit einem der Ringe (1, 2) durch Klemmung fest verbindbare Fixationsklammer (14), wobei diese einen im wesentlichen in der Knochenhauptachse (5) ausgerichteten Stab (15) aufnimmt, dessen freies Ende mit einer Fixationsvorrichtung (16) für einen senkrecht zur Knochenhauptachse (5) orientierten, den Knochen (8) haltenden Lenkdraht (17) versehen ist,
wobei die Vorrichtung durch folgende Merkmale gekennzeichnet ist:
- das jeweilige Verbindungselement (13) ist durch ein Spannschloß (49) und zwei in dieses eingeschraubte Spindeln (15), deren jeweils freies Ende einen Kugelansatz (47) aufweist, gebildet, wobei der jeweilige Kugelansatz (47) mit einer Kugelaufnahme (45) des jeweiligen Klammerelementes (11, 12) zusammenwirkt,
- das jeweilige Klammerelement (11, 12) weist ein U-förmig ausgebildetes Backenteil (33) und ein mit der Kugelaufnahme (34) versehenes Aufnahmeteil (35) auf,
- der Abstand der beiden Schenkel (36, 37) des Backenteils (33) entspricht der Stärke des zugeordneten Ringes (1, 2) in dessen axialer Erstreckung und es sind die beiden Schenkel (36, 37) im Bereich deren freien Enden miteinander verschraubt,
- das Aufnahmeteil (35) ist in der Knochenhauptachse (5) geteilt ausgebildet und es bildet eine (42) der Aufnahmeteilhälften (42,43) eine Baueinheit mit dem Backenteil (33) und ist mit der anderen Aufnahmeteilhälfte (43) verschraubt, wobei die Aufnahmeteilhälften (42, 43) auf den einander zugewandten Seiten die Kugelaufnahme (34) bildende Kugelhalbschalen (45) aufweisen, mit einer auf den anderen Ring (2, 1) zugerichteten Ausnehmung (46) zur Aufnahme der zugeordneten Kugel (47) und zum Durchtritt der zugeordneten Spindel (48) des jeweiligen längenveränderlichen Verbindungselementes (13).

2. Vorrichtung nach Anspruch 1, **dadurch gekennzeichnet,** daß eine Innensechskant schraube (38) die beiden Schenkel (36, 37) im Bereich deren freien Enden miteinander verbindet, sowie die beiden Aufnahmeteilhälften (42, 43) mittels zweier Innensechskantschrauben (44) verbunden sind.

3. Vorrichtung nach Anspruch 1 oder 2, **dadurch gekennzeichnet,** daß der jeweilige Ring (1, 2) im wesentlichen rechteckigen Querschnitt aufweist und dessen Hauptfläche im wesentlichen senkrecht zur Knochenhauptachse (5) angeordnet ist.

4. Vorrichtung nach Anspruch 1 oder 2, **dadurch gekennzeichnet,** daß der jeweilige Ring (1, 2) im wesentlichen rechteckigen Querschnitt aufweist und dessen Hauptfläche im wesentlichen in Knochenhauptachse (5) angeordnet ist.

5. Vorrichtung nach einem der Ansprüche 1 bis 4, **dadurch** **gekennzeichnet,** daß die Ringe (1, 2) unterschiedlichen Innendurchmesser bei im wesentlichen gleicher axialer und radialer Erstreckung aufweisen.

6. Vorrichtung nach einem der Ansprüche 1 bis 5, dadurch **gekennzeichnet,** daß die Ringe (1, 2) aus röntgenstrahlendurchlässigem Kunststoffmaterial bestehen.

7. Vorrichtung nach einem der Ansprüche 1 bis 6**, dadurch** **gekennzeichnet,** daß der jeweilige Ring (1, 2) zumindest in Teilumfangsbereichen mit einer Riffelung (66) versehen ist.

8. Vorrichtung nach einem der Ansprüche 1 bis 7, dadurch **gekennzeichnet,** daß der jeweilige Ring (1, 2) mehrteilig, insbesondere zweiteilig (1a, 1b) ausgebildet ist.

9. Vorrichtung nach einem der Ansprüche 1 bis 8, **dadurch** **gekennzeichnet,** daß jeder Ring (1, 2) mit vier Drahtklammern (6, 9) versehen ist, die jeweils um einen Winkel von 90° zueinander versetzt angeordnet sind.

10. Vorrichtung nach einem der Ansprüche 1 bis 9, **dadurch** **gekennzeichnet,** daß dem jeweiligen Ring (1, 2) in jeder seiner beiden Stirnseitenebenen ein Drahtkreuz zugeordnet ist.

11. Vorrichtung nach einem der Ansprüche 1 bis 10, **dadurch** **gekennzeichnet,** daß die jeweilige Drahtklammer (6, 7) U-förmig ausgebildet ist und der Abstand der beiden Schenkel (20, 21) der Drahtklammer (6, 9) der Stärke des zugeordneten Ringes (1, 2) in dessen axialer Erstreckung entspricht, sowie eine Innensechskantschraube (23) die beiden Schenkel (20, 21) im Bereich deren freien Enden miteinander verbindet, wobei zwischen dem Schraubenkopf (24) und dem zugeordneten Schenkel (20) eine Klemmplatte (26) zum Klemmen des dieser zugeordneten Fixierungsdrahtes (7, 10) zwischen der Klemmplatte (26) und dem Schenkel (20) vorgesehen ist, sowie die Klemmplatte (26) von der Schraube (23) durchsetzt wird.

12. Vorrichtung nach Anspruch 11, **dadurch gekennzeichnet**, daß die Drahtklammer (6, 9) nach radial außen auf den Ring (1, 2) aufsteckbar ist.

13. Vorrichtung nach einem der Ansprüche 1 bis 12, **dadurch** **gekennzeichnet,** daß das der Fixationsvorrichtung (16) abgewandte Ende des Lenkdrahtes (17) eine Verdickung (18) aufweist.

14. Vorrichtung nach einem der Ansprüche 1 bis 13, **dadurch** **gekennzeichnet,** daß zwei, insbesondere zueinander um 90° auf dem Ring (1, 2) versetzt angeordnete Fixationsklammern (14) vorgesehen sind, die jeweils mit der Fixationsvorrichtung (16) zum Halten eines die Verdickung (18) aufweisenden Lenkdrahtes (17) versehen sind.

15. Vorrichtung nach einem der Ansprüche 1 bis 14, **dadurch** **gekennzeichnet,** daß die Fixationsklammer (14) U-förmig ausgebildet ist und der Abstand der beiden Schenkel (56, 57) der Fixationsklammer (14) der Stärke des zugeordneten Ringes (1) in dessen axialer Erstreckung entspricht, sowie eine Innensechskantschraube (59) die beiden Schenkel (56, 57) im Bereich deren freien Enden miteinander verbindet.

16. Vorrichtung nach Anspruch 15, **dadurch gekennzeichnet,** daß die Fixationsklammer (14) im Bereich des die Schenkel (56, 57) verbindenden Steges mit einer Durchgangsbohrung in der Knochenhauptachse (5) versehen ist, die der über seine gesamte Länge mit einem Gewinde versehene Stab (15) durchsetzt, wobei zwei beabstandet der Bohrung angeordnete Muttern (55) den Gewindestab (15) mit der Fixationsklammer (14) verbinden.

17. Vorrichtung nach Anspruch 15, **dadurch gekennzeichnet,** daß die Fixationsklammer (14) zur Aufnahme des Stabes (15) ein Aufnahmeteil (88) aufweist, das in der Knochenhauptachse (5) geteilt ausgebildet ist, wobei eine der Aufnahmeteilhälften (90) eine Baueinheit mit dem Klammerteil der Fixationsklammer (14) bildet und mit der anderen Aufnahmeteilhälfte (91) mittels zweier Innensechskantschrauben (89) verbindbar ist, wobei ferner die Aufnahmeteilhälften (90, 91) auf den einander zugewandten Seiten eine Kugelaufnahme bildende Kugelhalbschalen aufweisen, mit einer in der Knochenhauptachse (5) verlaufenden Ausnehmung zur Aufnahme des eine Kugel (92) aufnehmenden eines Endes des Stabes (15), dessen anderes Ende mit einem Gewindeabschnitt versehen ist.

18. Vorrichtung nach Anspruch 16 oder 17, **dadurch gekenn****zeichnet,** daß auf den lagerfernen Gewindeabschnitt des Gewindestabes (15) die Fixationsvorrichtung (16) aufschraubbar ist, die aus einem auf den Gewindestab (15) aufschraubbaren Trägerelement (61), das einen Durchgang senkrecht zur Knochenhauptachse (5) aufweist und einer den Durchgang durchsetzenden Gewindehülse (62) zur Aufnahme des Lenkdrahtes (17) mit beidseitig des Trägerelementes (61) angeordneten Verstellmuttern (63) sowie einer in die Gewindehülse (62) einsetzbaren Konusklemmvorrichtung (64) für den Lenkdraht (17), besteht.

19. Vorrichtung nach einem der Ansprüche 1 bis 18, **dadurch** **gekennzeichnet,** daß ein Ende des jeweiligen Drahtes (7, 10, 17) mit einer ein Gewinde aufweisenden Spitze versehen ist.

## Claims

1. Apparatus for bone fixation, more particularly a bone of the human leg or arm, with the following features:
- at least two rings (1, 2) adapted to be spaced apart from one another and arranged perpendicular to the principal axis of the bone (5) and to be laid around the bone (8),
- wire braces (5, 6) adapted to be arranged on each ring (1, 2), distributed around the circumference thereof, and to be fixedly joined thereto by clamping, for holding fixation wires (7, 10) which pass right through the bone (8) in the manner of a cross,
- bracing elements (11, 12) adapted to be arranged on each ring (1, 2), distributed around the circumference thereof, and to be rigidly joined thereto by clamping, for flexibly receiving longitudinally adjustable connecting elements (13) which join the rings (1, 2),
- at least one fixation brace (14) adapted to be rigidly joined by clamping to one of the rings (1, 2), said fixation brace receiving a rod (15) which is aligned substantially in the principal bone axis (5) and the free end of which is provided with a fixation device (16) for a control wire (17) orientated perpendicular to the principal bone axis (5) and retaining the bone (8),
said apparatus being characterised by the following features:
- each respective connecting element (13) is constituted by a screw shackle (49) and two spindles (15) screwed thereinto, the respective free ends of which incorporate a ball-type lug (47), and each respective ball-type lug (47) cooperates with a ball locator (45) of the respective bracing element (11, 12), the respective bracing element (11, 12) incorporates a U-shaped jaw component (33) and a locating component (35) fitted with the ball locator (34), the spacing of the two legs (36, 37) of the jaw component (33) matches the thickness of the associated ring (1, 2) in the axial extension thereof, and the two legs (36, 37) are bolted together in the region of the free ends thereof,
- the locating component (35) is divided into two in the principal bone axis (5) and one (42) of the halves (42, 43) of the locating component forms a module with the jaw component (33) and is bolted to the other half (43) of the locating component, and the locating component halves (42, 43) incorporate on their mutually facing sides hemispherical dishes (45) which constitute the ball locator (34), with a recess (46) directed at the other ring (2, 1) for receiving the associated ball (47) and for passage of the associated spindle (48) of the respective longitudinally adjustable connecting element (13).

2. Apparatus according to claim 1, characterised in that a hexagon socket screw (38) joins together the two legs (36, 37) in the region of the free ends thereof, and the two halves of the locating component (42, 43) are connected by means of two hexagon socket screws (44).

3. Apparatus according to claim 1 or 2, characterised in that each respective ring (1, 2) is substantially rectangular in cross-section and the principal face thereof is disposed substantially perpendicular to the principal bone axis (5).

4. Apparatus according to claim 1 or 2, characterised in that each respective ring (1, 2) is substantially rectangular in cross-section and the principal face thereof is disposed substantially in the principal bone axis (5).

5. Apparatus according to any of claims 1 to 4, characterised in that the rings (1, 2) have different internal diameters but substantially the same axial and radial extension.

6. Apparatus according to any of claims 1 to 5, characterised in that the rings (1, 2) are made of a radiotranslucent plastic.

7. Apparatus according to any of claims 1 to 6, characterised in that each respective ring (1, 2) has a grooved surface (66) at least on parts of its circumference.

8. Apparatus according to any of claims 1 to 7, characterised in that each respective ring (1, 2) is constructed in more than one part, more particularly in two parts (1a, 1b).

9. Apparatus according to any of claims 1 to 8, characterised in that each ring (1, 2) is provided with four wire braces (6, 9) each offset at an angle of 90° with respect to one another.

10. Apparatus according to any of claims 1 to 9, characterised in that a wire cross is associated with each respective ring (1, 2) in both its end surface planes.

11. Apparatus according to any of claims 1 to 10, characterised in that each respective wire brace (6, 9) is U-shaped and the spacing between the two legs (20, 21) of the wire braces (6, 9) matches the thickness of the associated ring (1, 2) in the axial extension thereof, and a hexagon socket screw (23) joins together the two legs (20, 21) in the region of the free ends thereof, there being provided a clamping plate (26) between the screw head (24) and the associated leg (20) to clamp the fixation wire (7, 10) associated with said clamping plate (26) between the clamping plate (26) and the leg (20), and and the screw (23) passes right through the clamping plate (26).

12. Apparatus according to claim 11, characterised in that the wire brace (6, 9) is adapted to be pushed radially outwards onto the ring (1, 2).

13. Apparatus according to any of claims 1 to 12, characterised in that the end of the control wire (17) that faces away from the fixation device (16) has a thickened portion (18).

14. Apparatus according to any of claims 1 to 13, characterised in that two fixation braces (14) are provided, more particularly arranged on the ring (1, 2) and offset at 90° with respect to one another, each of said braces (14) being fitted with the fixation device (16) for retaining a control wire (17) incorporating the thickened portion (18).

15. Apparatus according to any of claims 1 to 14, characterised in that the fixation brace (14) is U-shaped and the spacing between the two legs (56, 57) of the fixation brace (14) matches the thickness of the associated ring (1) in the axial extension thereof, and a hexagon socket screw (59) joins together the two legs (56, 57) in the region of the free ends thereof.

16. Apparatus according to claim 15, characterised in that in the region of the crosspiece connecting the legs (56, 57) the fixation brace (14) is provided with a through-hole in the principal bone axis (5), and the rod (15), which is threaded along its entire length, passes right through said hole, while two nuts (55) spaced apart from the hole connect the threaded rod (15) to the fixation brace (14).

17. Apparatus according to claim 15, characterised in that to receive the rod (15) the fixation brace (14) incorporates a locating component (88) which is divided in the principal bone axis (5), one of the halves (90) of the locating component constituting a module with the clamping component of the fixation brace (14) and adapted to be joined to the other half (91) of the locating component by means of two hexagon socket screws (89), and in addition the halves (90, 91) of the locating component each incorporating on their facing sides a hemispherical dish forming a ball locator, with a recess extending in the principal axis (5) of the bone for receiving one end of the rod (15) which receives a ball (92), the other end of said rod (15) being provided with a threaded section.

18. Apparatus according to claim 16 or 17, characterised in that the fixation device (16) can be screwed onto the threaded section of the threaded rod (15) that is remote from its mounting, said fixation device (16) consisting of a supporting element (61) adapted to be screwed onto the threaded rod (15) and incorporating a passage perpendicular to the principal bone axis (5), and a threaded sleeve (62) which runs right through the passage to receive the control wire (17) with adjustment nuts (63) disposed on both sides of the supporting element (61) and with a cone clamping device (64) for the control wire (17), adapted for insertion into the threaded sleeve (62).

19. Apparatus according to any of claims 1 to 18, characterised in that the tip of one end of each wire (7, 10, 17) bears a thread.

## Revendications

1. Dispositif de fixation d'un os, en particulier d'un os de la jambe ou du bras d'un homme, dans lequel :
- au moins deux anneaux (1, 2) peuvent être placés, à une certaine distance l'un de l'autre, perpendiculairement à l'os (8) et autour de lui,
- sur chaque anneau (1, 2) peuvent être montées, réparties à volonté le long de sa périphérie et fixées par pincement, des pinces à fils (6, 9) servant de supports aux fils de fixation (7, 10) traversant en croix l'os (8),
- sur chaque anneau (1, 2) peuvent être répartis à volonté le long de sa périphérie et fixés par pincement, des éléments à pinces (11, 12) servant à recevoir, avec articulation, des éléments (13), de longueur variable, reliant les anneaux (1, 2),
- sur au moins un des anneaux (1, 2) peut être fixé, par pincement, une pince de fixation (14) recevant une barre (15) dirigée sensiblement selon l'axe principal (5) de l'os et dont l'extrémité libre est équipée d'un dispositif de fixation (16) portant selon une orientation perpendiculaire à l'axe principal (5) une tige (17) de maintien de l'os (8), ce dispositif présentant les caractéristiques suivantes :
- chaque élément de liaison (13) est constitué d'un tendeur (49) dans lequel sont vissées deux broches (15) dont les extrémités libres sont des rotules (47) montées dans un logement sphérique (34) des éléments à pince (11, 12) correspondant,
- chaque élément à pince (11, 12) présente une partie à mâchoires (33) en forme de U et une partie réceptrice (35) équipée du logement (34), la distance séparant les deux branches (36, 37) de la partie à mâchoires (33) correspondant à l'épaisseur, dans sa direction axiale, de l'anneau associé (1, 2), les extrémités libres des branches (36, 37) étant reliées par des vis,
- la partie réceptrice (35) est divisée en deux moitiés (42, 43) selon un plan parallèle à l'axe principal (5) de l'os, la moitié (42) constituant un ensemble avec la partie à mâchoires (33) et étant reliée par des vis à l'autre moitié (43),
- les moitiés (42, 43) présentent, sur leurs faces en regard, des demi-coquilles sphériques (45) constituant le logement (34) et ainsi que des évidements (46) dirigés vers l'autre anneau (2, 1), cet ensemble assurant le logement de la rotule (47) ainsi que le passage de la vis (48), faisant partie de l'élément longitudinal de liaison (13) associé.

2. Dispositif selon la revendication 1, caractérisé en ce que les deux branches (36, 37) sont reliées, à leurs extrémités, par une vis à six pans en creux (38), tandis que les deux moitiés (42, 43) de la pièce réceptrice sont reliées par une vis à six pans en creux (44).

3. Dispositif selon la revendication 1 ou 2, caractérisé en ce que chaque anneau (1, 2) a une section sensiblement rectangulaire et à sa face principale disposée sensiblement perpendiculairement à l'axe principal (5) de l'os.

4. Dispositif selon la revendication 1 ou 2, caractérisé en ce que chaque anneau (1, 2) a une section sensiblement rectangulaire et a sa face principale orientée sensiblement selon l'axe principal (5) de l'os.

5. Dispositif selon l'une des revendications 1 à 4, caractérisé en ce que les anneaux (1, 2) ont des diamètres internes différents, avec des dimensions axiales et radiales sensiblement égales.

6. Dispositif selon l'une des revendications 1 à 5, caractérisé en ce que les anneaux sont faits d'une matière plastique transparente aux rayons (Roëtgen).

7. Dispositif selon l'une des revendications 1 à 6, caractérisé en ce que chaque anneau (1, 2) porte, au moins sur certaines zones périphériques, des cannelures (66).

8. Dispositif selon l'une des revendications 1 à 7, caractérisé en ce que chaque anneau (1, 2) est réalisé en plusieurs parties, en particulier deux parties (1a, 1b).

9. Dispositif selon l'une des revendications 1 à 8, caractérisé en ce que chaque anneau (1, 2) est équipé de quatre pinces à fil (6, 9), réparties à 90° les unes des autres.

10. Dispositif selon l'une des revendications 1 à 9, caractérisé en ce que dans chaque anneau (1, 2), un fil est disposé en croix dans chacune de ses deux faces frontales.

11. Dispositif selon l'une des revendications 1 à 10, caractérisé en ce que chaque pince à fil (6, 7) a la forme d'un U dont les deux branches (20, 21) sont espacées de l'épaisseur axiale de l'anneau associé (1, 2) et reliées à leurs extrémités libres par une vis à six pans en creux (23), tandis qu'entre la tête (24) de cette vis et la branche (20) correspondante est intercalée une platine de pincement (26) servant à assurer le pincement du fil de fixation (7, 10) entre la branche (20) et la platine (26) que traverse la vis (23).

12. Dispositif selon la revendication 11, caractérisé en ce que la pince à fil (6, 9) peut être emboîtée sur l'anneau (1, 2), radialement de l'extérieur.

13. Dispositif selon l'une des revendications 1 à 12, caractérisé en ce que l'extrémité du fil d'orientation (17), opposée au dispositif de fixation (16) présente une surépaisseur (18).

14. Dispositif selon l'une des revendications 1 à 13, caractérisé en ce que sont prévues deux pinces de fixation (14) disposées décalées l'une par rapport à l'autre de 90° sur l'anneau (1, 2) dont chacune porte le dispositif de fixation (16) servant à maintenir un fil d'orientation (17) présentant une surépaisseur (18).

15. Dispositif selon l'une des revendications 1 à 14, caractérisé en ce que la pince de fixation (14) a la forme d'un U dont les deux branches (56, 57) de la pince de fixation (14) sont espacées de l'épaisseur axiale de l'anneau associé (1) et reliées à leurs extrémités par une vis à six pans en creux (59).

16. Dispositif selon la revendication 15, caractérisé en ce que la pince de fixation (14) et percée, dans la zone de jonction des branches (56, 57), d'un alésage parallèle à l'axe principal de l'os (5) et dans lequel est vissée une tige (15) filetée sur toute sa longueur et bloquée sur la pince de fixation (14) par deux écrous (55) situés de part et d'autre de l'alésage.

17. Dispositif selon la revendication 15, caractérisé en ce que la pince de fixation (14) est équipée, pour loger la tige (15), d'une partie réceptrice (88), divisée en deux moitiés selon un plan parallèle à l'axe principal de l'os (5), la moitié (90) constituant un ensemble avec la partie à pince de fixation (14) tandis que l'autre moitié (91) peut être reliée à la première par deux vis à six pans en creux (89), les moitiés (90, 91) étant équipées, sur les faces en regard, de demi-coquilles sphériques délimitant un logement sphérique et présentant chacune un évidement selon la direction de l'axe principal de l'os (5), de sorte que le logement permet de recevoir une rotule (92) montée à l'extrémité de la tige (15) dont l'autre extrémité est équipée d'un filetage.

18. Dispositif selon la revendication 16 ou 17, caractérisé en ce qu'un dispositif de fixation (16) peut être vissé sur la partie filetée, opposée à son palier, de la tige (15), ce dispositif de fixation étant constitué d'un support (61) vissable sur la tige (15), percé d'un alésage perpendiculaire à l'axe principal de l'os (5) et dans lequel est vissée une douille (62) que traverse le fil d'orientation (17) et qui est encadrée par deux écrous de réglage (63), un dispositif à pincement conique (64), retenant le fil d'orientation (17) pouvant être monté dans la douille filetée (62).

19. Dispositif selon l'une des revendications 1 à 18, caractérisé en ce que l'extrémité de chaque fil (7, 10, 17) est équipée d'une pointe portant un filetage.
